# EUROPEAN PATENT SPECIFICATION

(11) **EP 1 834 591 B1**
(45) Date of publication and mention of the grant of the patent: **13.05.2009**
(21) Application number: 07005243.6
(22) Date of filing: 14.03.2007
(51) Int. Cl.: A61B 17/04

(54) **Suture pushing apparatus**
Schiebevorrichtung für Wundnähte
Appareil pour pousser les sutures

(30) Priority: 15.03.2006 US 375960
(43) Date of publication of application: 19.09.2007
(73) Proprietor: United States Surgical Corporation, North Haven, CT 06473 (US)
(72) Inventor: Bayer, Hanspeter Robert, Meriden CT 06451-2840 (US); Martinek, Jonathan, Cheshire CT 06410 (US)
(74) Representative: HOFFMANN EITLE

(56) References cited:
- JP-A- 5 317 321
- US-A- 2 595 086
- US-A- 5 133 723
- US-A- 5 759 189
- US-A1- 2001 041 901

## Description

### BACKGROUND

### 1. Field of the Disclosure

The present disclosure relates generally to surgical instruments. In particular, the present disclosure relates to a suture or suture knot pushing apparatus used during arthroscopic or similar surgical procedures.

### 2. Description of the Related Art

Minimally invasive procedures have several advantages over traditional open surgery, including less patient trauma, reduced recovery time, and reduced potential for infection. However, despite its recent success and overall acceptance as a preferred surgical technique, minimally invasive surgery, such as laparoscopy or arthroscopy, has several disadvantages. For example, surgery of this type, particularly, arthroscopic surgical procedures, often requires the placement of sutures within the body and subsequent tying of a suture knot in an area, e.g., a ligament or tendon, which may be difficult to access.

A number of methods have been developed to assist surgeons in the tying of these suture knots. One method involves the tying of sutures directly within the body, i.e., intracorporeal suture knot tying, a procedure which is often very difficult because of spatial constraints. A second method, extracorporeal suture knot tying, includes forming the suture knot outside of the body and then transferring or "running" the suture knot to the desired tissue location inside the body.

Some conventional extracorporeal suture knot tying methodologies incorporate a suture knot pusher or runner to advance the suture knot to the targeted tissue site. With these devices, a throw is formed outside of the body in the two free ends of the suture. Then, the throw is positioned on the shaft of the suture knot pusher. Once the throw is seated, the surgeon must carefully attempt to transfer the throw into the body and directly to the surgical site where it will be secured adjacent the desired tissue.

Conventional suture knot pusher devices have significant limitations. Many of these instruments are ill equipped to permit an equal level of tension to be placed on each end of the suture. If the proper amount of tension is not imparted to the suture ends, the suture knot may become loose and the tissue may not be properly secured. In addition, the suture knot may have a tendency to slide off the leading end of the suture knot pusher, making it extremely difficult to effectively secure the tissue. Moreover, it is practice during some arthroscopic procedures to inject high pressure fluid into the body. This can cause a myriad of problems as the hollow shaft of many existing suture knot pushers acts as a conduit for the fluid to spew, thus possibly contaminating the surgical site and/or interfering with the procedure.

US 2001/0041901 discloses a suture pushing apparatus having the features of the preamble of claim 1.

US 5,133,723 discloses a suture throw rundown tool including an elongate shaft and a means for engaging the respective throws as they are run down the suture ends. The means being a groove extending transversely across the front face or a depression on the front end face.

JP 5317321 discloses a clipper for pushing a knot, in a filament, made outside the body to be smoothly shifted into the interior of the body and to ensure clipping of a blood vessel. The clipper has a recessed part for guiding a clipping part of the filaments at the forward end.

### SUMMARY

Accordingly, the present disclosure is directed to a suture pushing apparatus adapted for effectively advancing a suture knot relative to a tissue site. The suture pushing apparatus, and this is recited in the sole independent claim. Claim 1, includes an elongated shaft defining proximal and distal ends, and having a suture pushing member adjacent the distal end thereof. The suture pushing member includes first and second retainer members extending in a general longitudinal direction relative to a longitudinal axis defined by the elongated shaft and being disposed in lateral spaced relation with respect to each other for reception of a suture knot therebetween. The first and second retainer members each include a recess for receiving respective suture lengths extending from the suture knot and are dimensioned for retaining the suture lengths within confines of the recesses. First and second passages are defined in the elongated shaft adjacent the respective first and second retainer members and are in communication with an internal longitudinal passageway of the elongated shaft whereby the first and second passages receive suture lengths respectively extending from the first and second retainer members for passage through the longitudinal passageway and toward a surgeon. The elongated shaft may be generally solid adjacent the proximal end thereof to prevent fluid flow toward the surgeon. The elongated shaft may further include an internal surface proximal of the longitudinal passageway. The internal surface is adapted to guide the suture ends from the longitudinal passageway to a location external of the elongated shaft. The internal surface may be obliquely arranged relative to the longitudinal axis of the elongated shaft.

The first and second retainer members may be laterally spaced to define a gap therebetween for accommodating the suture knot in suspended relation relative to the suture pushing member. In one embodiment, the elongated shaft includes first and second apertures extending through a wall of the elongated shaft and defining the first and second passages. Alternatively, the elongated shaft may include first and second grooves defined in a wall portion of the elongated shaft. The first and second grooves are the first and second passages.

Another suture pushing apparatus, which does not form part of the invention disclosed herein includes an elongated shaft defining proximal and distal ends, and having a suture pushing member adjacent the distal end thereof. The suture pushing member includes first and second retainer members disposed in lateral spaced relation for reception of a suture knot therebetween. The first and second retainer members each include a recess for receiving respective suture lengths extending from the suture knot to maintain the suture knot in suspended relation. First and second passages are defined in the elongated shaft in communication with the recesses of the respective first and second retainer members for receiving the suture lengths extending from the first and second retainer members for passage in a general proximal direction toward a surgeon. The elongated shaft may include first and second longitudinal grooves defined in the periphery thereof. The first and second longitudinal grooves define the first and second passages. The first and second longitudinal grooves extend substantially along the length of the elongated shaft.

### BRIEF DESCRIPTION OF THE DRAWINGS

Preferred embodiments of the present disclosure will be better appreciated by reference to the drawings wherein:
FIG. 1 is a perspective view of the suture pushing apparatus in accordance with the principles of the present disclosure;
FIG. 2 is an enlarged perspective view illustrating the suture pusher member of the suture pushing apparatus;
FIG. 3 is a perspective view illustrating a suture knot positioned relative to the suture pushing member of the suture pushing apparatus;
FIG. 4 is a perspective view further illustrating the suture knot and suture ends positioned relative to the suture pushing apparatus;
FIG. 5 is a perspective view an alternate embodiment of the suture pushing apparatus of the present disclosure;
FIG. 6 is a perspective view of another alternate embodiment of the suture pushing apparatus of the present disclosure;
FIG. 7 is an enlarged perspective view illustrating the suture pusher member of the suture pushing apparatus of FIG. 6; and
FIG. 8 is a perspective view illustrating a suture pushing apparatus, which does not form part of the invention.

### DETAILED DESCRIPTION OF THE PREFERRED EMBODIMENTS

The suture pushing apparatus of the present disclosure is intended to be used during minimally invasive surgical procedures to provide a viable option to surgeons who are unable to tie a suture knot directly at the affected area. Moreover, the present disclosure enables the surgeon to simultaneously apply equal tension to each side of a suture while the suture knot is suspended in its desired position, therefore ensuring that the suture knot is uniformly tightened against the tissue.

As those skilled in the art will appreciate, it is common practice during these procedures to inject highly pressurized fluid into the body. In one preferred embodiment of the present disclosure, the suture pushing apparatus is particularly designed to prevent this high pressure fluid from traveling back out of the body and distracting the surgeon. The apparatus also may be adapted to receive a surgical instrument such as a fluid injecting instrument or a visualization device.

In the following description, as is traditional, the term "proximal" refers to the portion of the apparatus closest to the operator, while the term "distal" refers to the portion of the apparatus remote from the operator.

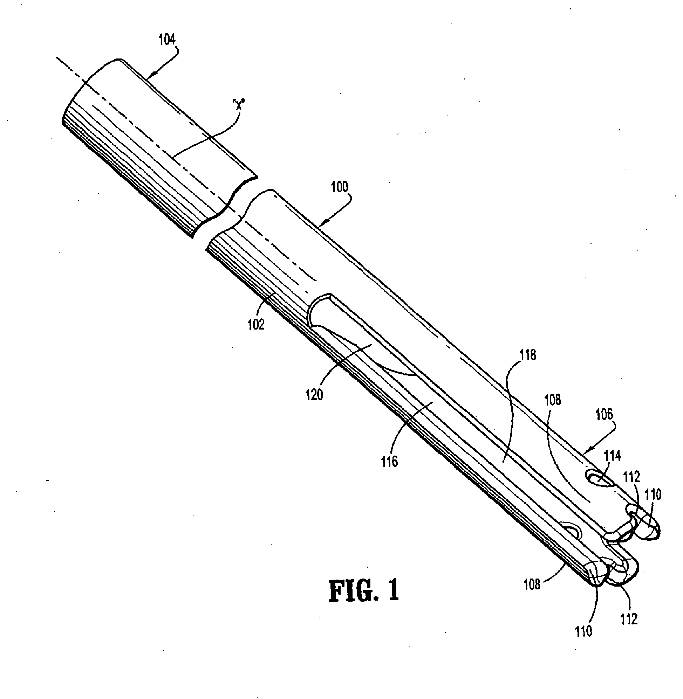

Referring now to the drawings, in which like reference numerals identify identical or substantially similar parts throughout the several views, FIG. 1 illustrates, in perspective view, the suture pushing apparatus 100 of the present disclosure. Suture pushing apparatus 100 is configured to capture a suture knot and retain the suture knot in a suspended and fixed position relative to the apparatus 100 while the suture knot is advanced toward the surgical site. Suture pushing apparatus 100 may be constructed of a plurality of different materials including but not limited to stainless steel, titanium and/or alloys thereof, and polymeric materials. Suture pushing apparatus 100 may be disposable after use or reusable. If reusable, suture pushing apparatus 100 may be sterilized for subsequent use.

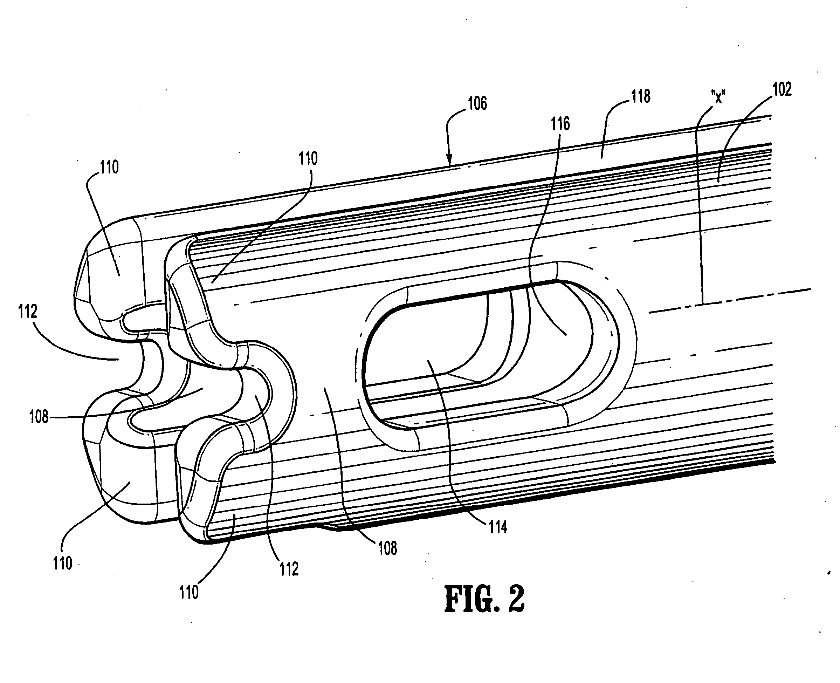

Referring now to FIGS. 1-2, suture pushing apparatus 100 includes elongated shaft 102 having longitudinal axis "x" and proximal and distal ends 104, 106. Preferably, proximal end 104 of the shaft 102 is substantially solid in structure to prevent fluid from flowing back through the shaft 102 during high pressure fluidization procedures. Alternatively, proximal end 104 may include a longitudinal bore therethrough as will be discussed hereinbelow. Distal end 106 of elongated shaft 102 incorporates the suture knot retainer/pusher member of the apparatus 100. Specifically, distal end 106 is bifurcated defining first and second retainer members 108 which are laterally spaced with respect to the longitudinal axis "x". Each retainer member 108 includes a pair of spaced protrusions 110 separated by arcuate recess 112. Protrusions 110 are dimensioned to retain a suture portion received within arcuate recess 112. Protrusions 110 define arcuate or rounded corners which are preferably atraumatic to minimize potential of slicing and/or cutting of a suture when positioning the suture relative to distal end 106. Arcuate recess 112 is preferably sufficient in depth to receive and accommodate a suture portion and, in combination with protrusions 110, prevent inadvertent removal of the suture portion therefrom. Arcuate recesses 112 are also dimensioned to permit the suture to traverse the recesses 112 during tightening and securement of the suture.

Disposed proximal of retainer members 108 are a pair of diametrically opposed apertures 114 extending through the wall of elongated shaft 102. Apertures 114 receive suture lengths extending from the suture knot and from retainer members 108.

Shaft 102 further includes central passage or channel 116 which extends along the longitudinal axis "x", and longitudinal slot 118 defined in the outer wall surface of the elongated shaft 102 in communication with the central channel 116. Central channel 116 establishes a passageway for the two suture ends received through apertures 114. Central channel 116 extends to an intermediate location of shaft 102 and terminates at internal ramp 120. Internal ramp 120 is obliquely arranged relative to longitudinal axis "x". Internal ramp 120 is adapted to guide the suture ends out of central channel 116 through longitudinal slot 118 where the suture ends are then passed back toward proximal end 104 of shaft 102. Internal ramp 120 also functions in directing the pressurized fluids in a lateral direction away from the physician. Longitudinal slot 118 permits access to central channel 116 to assist in loading and/or manipulating the suture within suture pushing apparatus 100.

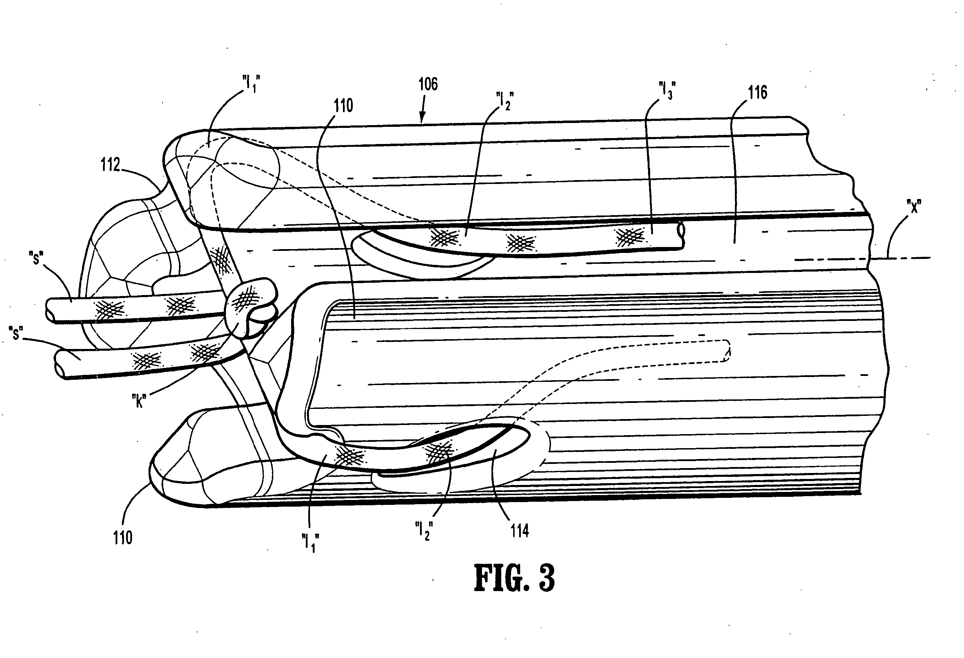

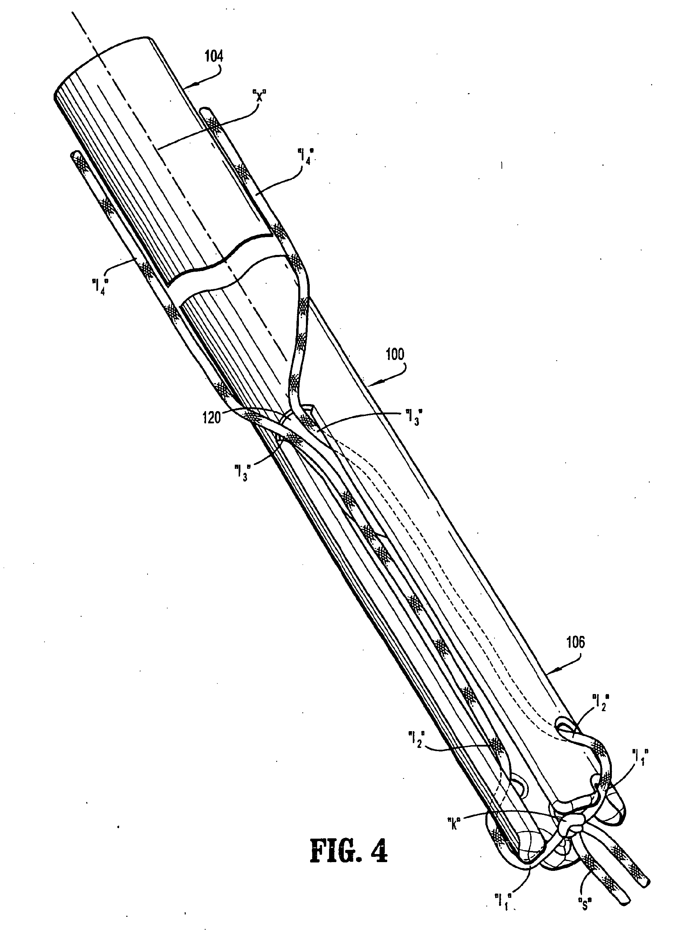

Referring now to FIGS. 3-4, the use of suture pushing apparatus 100 in an endoscopic procedure will be discussed. A suture "s" is stitched through the desired tissue, e.g., through a tendon, ligament, muscle or bone. The two ends of the suture "s" are extended outside of the body in order to form the suture knot(s) extracorporeally. The suture knot "k", preferably, a slip suture knot, is formed and mounted to retainer members 108 in suspended manner. More specifically, the suture knot "k" is positioned within the space defined between retainer members 108. The suture lengths "l₁" which extend immediately from the suture knot "k" are received within arcuate recesses 112 of retainer members 108. Thereafter, the suture lengths "l₂" extending immediately from the suture length "l₁" are laterally passed through respective apertures 114 of first and second retainer members 108 to enter central channel 116. The suture lengths "l₃" are arranged to extend within central channel 116 toward proximal end 104 and are subsequently guided out of central channel 116 and through longitudinal slot 118 along internal ramp 120. The suture lengths or ends "l₄" are passed along the exterior of elongated shaft 102 toward the surgeon.

Once the suture "s" and the suture knot "k" are positioned relative to apparatus 100 in the aforedescribed manner, the surgeon can focus on securing the suture knot "k" relative to the tissue. The suture ends "l₄" are grasped by the surgeon with one hand. While holding the suture ends "l₄" with the one hand, the surgeon advances the suture pushing apparatus 100 toward the tissue site with the other hand. Suture pushing apparatus 100 is moved forward thereby causing the distal end 106 to engage the suture lengths "l₁" adjacent the suture knot "k" to force the suture knot "k" in the distal direction toward the tissue. As the suture knot "k" is driven toward the tissue site, the suture ends traverse arcuate recesses 112 of retainer members 108. By virtue of engagement of retainer members 108 with the suture lengths "l₁" adjacent the suture knot "k" (and not the suture knot "k" itself), an even amount of tension is applied to each end of the suture thus ensuring uniform securement of the suture knot "k" against tissue. Once the suture knot "k" has been secured, the operation may be repeated in order to apply additional sutures. Suture pushing apparatus 100 may be retracted or removed, leaving the suture knot "k" tightly in place. As appreciated, in the presence of high pressurized fluids, the solid proximal end 104 prevents the passage of fluids toward the surgeon. Moreover, the fluid may be laterally directed away from the longitudinal axis "x" of the apparatus 100 and the surgeon via internal ramp 120.

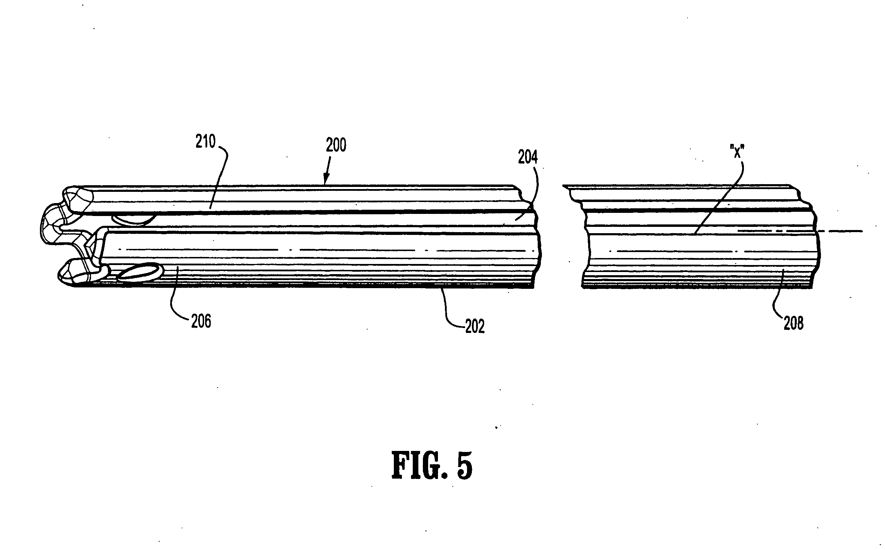

Referring now to FIG. 5, an alternate embodiment of the suture pushing apparatus 200 is disclosed. In accordance with this embodiment, elongated shaft 202 includes central channel 204 which extends from distal end 206 of the shaft 202 along the longitudinal axis "x" completely through proximal end 208. With this arrangement, it is envisioned that a surgical instrument could be introduced through central channel 204 to facilitate the performances of the surgical procedure. For example, a laparoscope may be introduced through central channel 204 to permit visualization prior to loading of the suture "s". Elongated shaft 202 also includes longitudinal slot 210 in communication with central channel 204 to permit access to the central channel 204 to assist in positioning the suture within the central channel 204 of the elongated shaft 202. Longitudinal slot 210 may extend the length of elongated shaft 202. In use of this embodiment, the suture ends or lengths "l₄" do not exit an intermediate portion of elongated shaft 202 as in the embodiment of FIGS. 1-4, but, remain within central channel 204 of the elongated shaft 202 to extend from proximal end 208 through the central channel 204. In other respects, the suture knot "k" is secured relative to the tissue in a similar manner to that described in connection with the embodiment of FIGS. 1-4.

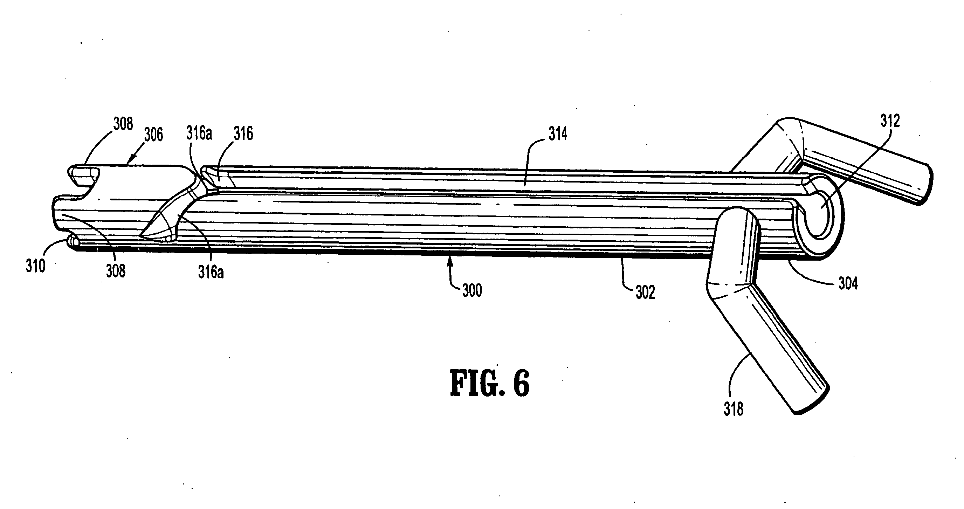

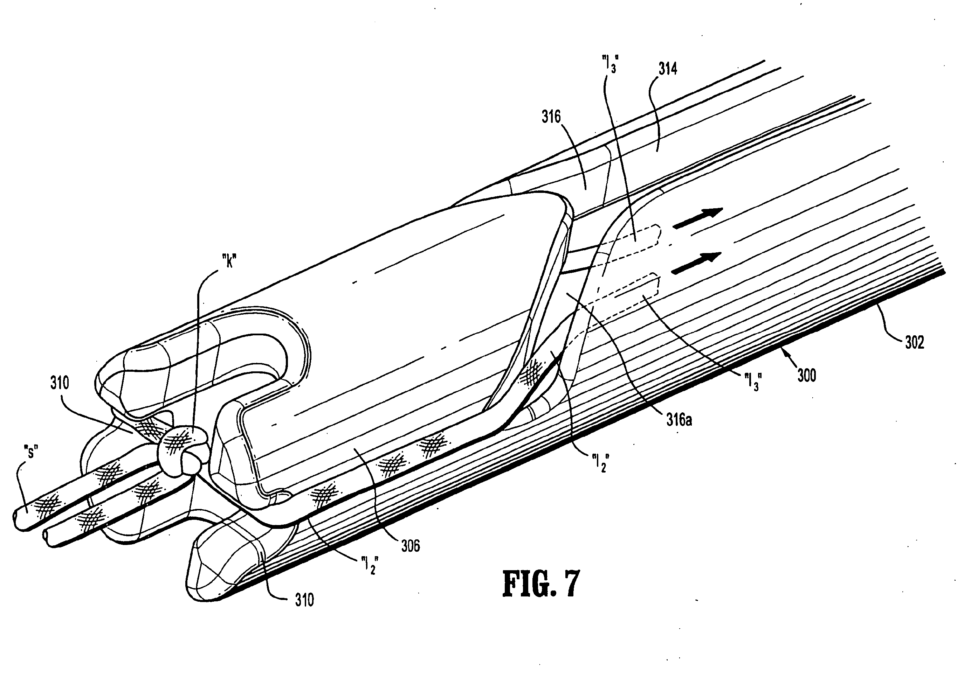

FIGS. 6-7 illustrate an alternate embodiment of the present disclosure. Suture pushing apparatus 300 includes elongated shaft 302 having proximal end 304 and distal end 306. Distal end 306 includes diametrically opposed retainer members 308 which are spaced to receive the suture knot "k". Retainer members 308 define arcuate recesses 310 for receiving the suture lengths "l₁" and to maintain the suture knot "k" in suspended relation relative to the retainer members 308. Elongated shaft 302 further includes central channel 312 extending completely through proximal end 304. Central channel 312 is open, i.e., elongated shaft 302 has longitudinal slot 314 which communicates with the central channel 312 and extends to the exterior of elongated shaft 302. Central channel 312 and longitudinal slot 314 terminate at "y" shaped groove 316 formed in the outer surface of the elongated shaft adjacent distal end 306. "Y' shaped groove 316 includes groove portions 316a which are longitudinally aligned with respective arcuate recesses 310 of retainer members 308 to receive the suture lengths "l₂" extending from the arcuate recesses 310. Groove portions 316a are preferably devoid of sharp surfaces to minimize the potential of suture fraying or tearing. The remaining portion of distal end 306 proximal of retainer members 308 may be solid if desired. Central channel 312 receives the suture lengths "l₃" which are routed back to the surgeon. Suture pushing apparatus 300 also includes handle 314 to facilitate manipulation and enhance control of the apparatus 300. Handle 314 may be of any suitable ergonomic design. Suture pushing apparatus 300 is used in a similar manner to the embodiments of FIGS. 1-4 and FIG. 5.

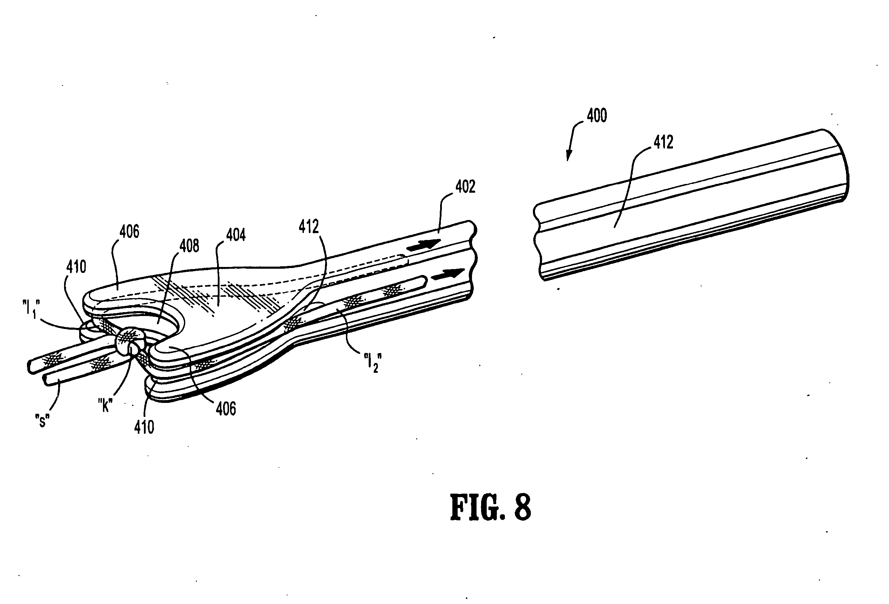

Referring now to FIG. 8, (a) suture pushing apparatus, which does not form part of the invention, is disclosed. Note that claim 1 requires first and second passages defined in the elongated shaft and in communication with an internal longitudinal passageway of the elongated shaft. Suture pushing apparatus 400 does not include such passages. In accordance with this, suture pushing apparatus 400 includes elongated shaft 402 having suture pusher head 404 adjacent the distal end of the elongated shaft 402. Suture pusher head 404 defines a general U-shape in plan view and incorporates first and second opposed retainer members 406 extending in a general longitudinal direction. Retainer members 406 are laterally spaced to define gap 408 for accommodating the suture knot "k". Retainer members 406 each define arcuate recess 410 for receiving the suture lengths "11" adjacent the suture knot "k" and to maintain the suture knot "k" in a suspended relationship relative to suture pusher head 404. Suture pusher head 406 defines a rectangular appearance or cross-section which may present a reduced profile thereby facilitating position or advancement of the suture pushing apparatus 400 relative to the tissue.

Suture pusher apparatus 400 includes a pair of longitudinal grooves 412 extending along the peripheries of suture pusher head 404 and along elongated shaft 402. Longitudinal grooves 412 are in communication with respective arcuate recesses 410 of suture pusher head 404 and receive the suture ends or lengths "12" extending from the arcuate recesses 402. Longitudinal grooves 412 preferably extend the length of elongated shaft 402 completely through the proximal end of the elongated shaft 402; however, it is envisioned that the longitudinal grooves 412 may terminate in a ramped or inclined surface which guides the suture ends along the exterior of the proximal end of the elongated shaft 402. Longitudinal grooves 412 are configured to contain the suture ends therein while permitting advancement of the suture pushing apparatus 400 relative to the suture "s". In use, the suture "s" is applied to the tissue and the suture knot "k" is formed extracorporally in the same manner as described in the prior embodiments. The suture knot "k" is mounted relative to suture pusher head 404 in suspended manner as depicted in FIG. 8 with the suture lengths "l₁" received within arcuate recesses 410 of suture pusher head 404. The surgeon then positions the suture lengths "l₂" within longitudinal grooves 412 and routes the suture ends " l₂" back toward the proximal end and toward the surgeon. The surgeon can thereafter secure the suture knot "k" relative to the tissue by advancing the suture pusher apparatus 400 relative to the suture "s" while applying equal tension to the suture ends "l₂".

It will be understood that various modifications and changes in form and detail may be made to the embodiments of the present disclosure without departing from the scope of the invention. Therefore, the above description should not be construed as limiting the invention but merely as exemplifications of preferred embodiments thereof.

## Claims

1. A suture pushing apparatus (100, 200, 300), comprising:
an elongated shaft (102, 202, 302) defining proximal, and distal ends,
having a suture pushing member (106) adjacent the distal end thereof,
the suture pushing member includes first and second retainer members (108, 308) extending in a general longitudinal direction relative to a longitudinal axis defined by the elongate shaft (102, 202, 302), and being disposed in lateral spaced relation with respect to each other for reception of a suture knot therebetween,
the first and second retainer members each including a recess (112, 310) for receiving respective suture lengths extending from the suture knot and being dimensioned for retaining the suture lengths within confines of the recesses, and
having first and second passages (114, 316) defined in the elongated shaft (102, 202, 302) adjacent respective first and second retainer members (108, 308), and
**characterised in that** the elongated shaft (102, 202, 302) further comprises an internal longitudinal passageway (116, 204, 312) in communication with said first and second passages (114, 136), whereby the first and second passages (114, 316) receive suture lengths respectively extending from the first and second retainer members (108, 308) for passage through the longitudinal passageway (116, 204, 312) and toward a surgeon.

2. The suture pushing apparatus according to claim 1, wherein the elongated shaft (102, 202, 302) is generally solid adjacent the proximal end thereof.

3. The suture pushing apparatus according to claim 1 or 2, wherein the elongated shaft (102, 202, 302) includes an internal surface (120) proximal of the longitudinal passageway (116, 204, 312), the internal surface adapted to guide the suture ends from the longitudinal passageway to a location external of the elongated shaft.

4. The suture pushing apparatus according to claim 3, wherein the internal surface is obliguely arranged relative to the longitudinal axis of the elongated shaft.

5. The suture pushing apparatus according to any one of the preceding claims, wherein the first and second retainer members are laterally spaced to define a gap (408) therebetween for accommodating the suture knot in suspended relation relative to the suture pushing member.

6. The suture pushing apparatus according to any one of the preceding claims, wherein the elongated shaft includes first and second apertures (114) extending through a wall of the elongated shaft and defining the first and second passages.

7. The suture pushing apparatus according to any one of claims 1 to 5, wherein the elongated shaft includes first and second grooves (316) defined in a wall portion of the elongated shaft, the first and second grooves being the first and second passages.

8. The suture pushing apparatus according to any one of the preceding claims, further comprising a handle (318) affixed to the proximal end of the elongated shaft.

9. The suture pushing apparatus according to any one of the preceding claims, wherein the elongated shaft includes a longitudinal bore (312) for reception of a surgical instrument.

10. The suture pushing apparatus according to claim 9, wherein the longitudinal bore is the longitudinal passageway of the elongated shaft.

## Patentansprüche

1. Fadenstoßvorrichtung (100, 200, 300), umfassend
einen länglichen Schaft (102, 202, 302), der proximale und distale Enden definiert,
mit einem an sein distales Ende anschließenden Fadenstoßelement (106),
wobei das Fadenstoßelement erste und zweite Haltemittel (108, 308) aufweist, die sich im Wesentlichen längs relativ zu einer Längsachse erstrecken, die durch den länglichen Schaft (102, 202, 302) definiert ist, und in einer seitlich beabstandeten Beziehung in Bezug aufeinander zur Aufnahme eines Fadenknotens dazwischen angeordnet sind,
wobei die ersten und zweiten Haltemittel jeweils eine Aussparung (112, 310) zum jeweiligen Aufnehmen von Fadenlängen enthalten, die sich von dem Fadenknoten erstrecken und zum Beibehalten der Fadenlängen innerhalb der Abgrenzungen der Aussparungen bemessen sind, und
mit ersten und zweiten Durchgängen (114, 316), die in dem länglichen Schaft (102, 202, 302) benachbart zu entsprechenden ersten und zweiten Halteelementen (108, 308) definiert sind, und
**dadurch gekennzeichnet, dass** der längliche Schaft (102, 202, 302) ferner einen inneren Längsdurchgang (116, 204, 312) in Verbindung mit den ersten und zweiten Durchgängen (114, 136) aufweist, durch die die ersten und zweiten Durchgänge (114, 316) die Fadenlängen aufnehmen, die sich jeweils von den ersten und zweiten Halteelementen (108, 308) zum Durchgang durch den Längsdurchgang (116, 204, 312) und in Richtung eines Chirurgen erstrecken.

2. Fadenstoßvorrichtung nach Anspruch 1, wobei der längliche Schaft (102, 202, 302) benachbart zu seinem proximalen Ende im Allgemeinen fest ist.

3. Fadenstoßvorrichtung nach Anspruch 1 oder 2, wobei der längliche Schaft (102, 202, 302) eine innere Oberfläche (120) enthält, die proximal von dem Längsdurchgang (116, 204, 312) ist, wobei die innere Oberfläche dazu angepasst ist, die Fadenenden von dem Längsdurchgang zu einem Ort außerhalb des länglichen Schafts zu führen.

4. Fadenstoßvorrichtung nach Anspruch 3, wobei die innere Oberfläche relativ zu der Längsachse des länglichen Schafts schräg angeordnet ist.

5. Fadenstoßvorrichtung nach einem der vorstehenden Ansprüche, wobei das erste und zweite Halteelement seitlich beabstandet sind, um eine Lücke (408) dazwischen zum Aufnehmen des Fadenknotens in hängender Beziehung relativ zu dem Fadenstoßelement zu definieren.

6. Fadenstoßvorrichtung nach einem der vorstehenden Ansprüche, wobei der längliche Schaft erste und zweite Öffnungen (114) enthält, die sich durch eine Wand des länglichen Schafts erstrecken und die ersten und zweiten Durchgänge definieren.

7. Fadenstoßvorrichtung nach einem der Ansprüche 1 bis 5,
wobei der längliche Schaft erste und zweite Nuten (316) enthält, die in einem Wandabschnitt des länglichen Schafts definiert sind, wobei die ersten und zweiten Nuten die ersten und zweiten Durchgänge sind.

8. Fadenstoßvorrichtung nach einem der vorstehenden Ansprüche, ferner umfassend einen Griff (318), der an dem proximalen Ende des länglichen Schafts befestigt ist.

9. Fadenstoßvorrichtung nach einem der vorstehenden Ansprüche, wobei der längliche Schaft eine Längsbohrung (312) zur Aufnahme eines chirurgischen Instruments enthält.

10. Fadenstoßvorrichtung nach Anspruch 9, wobei die Längsbohrung der Längsdurchgang des länglichen Schafts ist.

## Revendications

1. Appareil de poussée de matériau de suture (100, 200, 300), comprenant:
un arbre oblong (102, 202, 302) définissant des extrémités proximale et distale,
ayant un élément de poussée de matériau de suture (106) adjacent à son extrémité distale,
l'élément de poussée de matériau de suture comprend des premier et second éléments de retenue (108, 308) s'étendant dans une direction généralement longitudinale relativement à un axe longitudinal défini par l'arbre oblong (102, 202, 302) et étant disposé dans une relation latérale espacée l'un par rapport à l'autre pour la réception d'un noeud de matériau de suture entre eux,
les premier et second éléments de retenue ayant chacun un évidement (112, 310) pour recevoir des longueurs de suture respectives s'étendant depuis le noeud de suture et étant dimensionnés pour retenir les longueurs de suture dans le confinement des évidements, et
ayant des premier et second passages (114, 316) définis dans l'arbre oblong (102, 202, 302) adjacents aux premier et second éléments de retenue respectifs (108, 308), et
**caractérisé en ce que** l'arbre oblong (102, 202, 302) comprend en outre un passage longitudinal interne (116, 204, 312) en communication avec lesdits premier et second passages (114, 136), par quoi les premier et second passages (114, 316) reçoivent des longueurs de suture s'étendant respectivement des premier et second éléments de retenue (108, 308) pour le passage à travers le passage longitudinal (116, 204, 312) et vers un chirurgien.

2. Appareil de poussée de matériau de suture selon la revendication 1, où l'arbre oblong (102, 202, 302) est généralement plein adjacent à son extrémité proximale.

3. Appareil de poussée de matériau de suture selon la revendication 1 ou 2, où l'arbre oblong (102, 202, 302) comprend une surface interne (120) proximale du passage longitudinal (116, 204, 312), la surface interne étant apte à guider les extrémités de suture du passage longitudinal vers un emplacement à l'extérieur de l'arbre oblong.

4. Appareil de poussée de matériau de suture selon la revendication 3, où la surface interne est agencée en biais relativement à l'axe longitudinal de l'arbre oblong.

5. Appareil de poussée de matériau de suture selon l'une quelconque des revendications précédentes, où les premier et second éléments de retenue sont espacés latéralement pour définir un espace (408) entre eux pour recevoir le noeud de suture en une relation suspendue relativement à l'élément de poussée de matériau de suture.

6. Appareil de poussée de matériau de suture selon l'une quelconque des revendications précédentes, où l'arbre oblong comprend des première et seconde ouvertures (114) s'étendant à travers une paroi de l'arbre oblong et définissant les premier et second passages.

7. Appareil de poussée de matériau de suture selon l'une quelconque des revendications 1 à 5, où l'arbre oblong comprend des première et seconde rainures (316) définies dans une portion de paroi de l'arbre oblong, les première et seconde rainures étant les premier et second passages.

8. Appareil de poussée de matériau de suture selon l'une quelconque des revendications précédentes, comprenant en outre une poignée (318) fixée à l'extrémité proximale de l'arbre oblong.

9. Appareil de poussée de matériau de suture selon l'une quelconque des revendications précédentes, où l'arbre oblong comprend un perçage longitudinal (312) pour la réception d'un instrument chirurgical.

10. Appareil de poussée de matériau de suture selon la revendication 9, où le perçage longitudinal est le passage longitudinal de l'arbre oblong.
